# EUROPEAN PATENT SPECIFICATION

(11) **EP 2 854 931 B1**
(45) Date of publication and mention of the grant of the patent: **06.07.2016**
(21) Application number: 13735428.8
(22) Date of filing: 17.05.2013
(51) Int. Cl.: A61M 39/10

(54) **CONNECTION DEVICE**
VERBINDUNGSVORRICHTUNG
DISPOSITIF DE RACCORDEMENT

(30) Priority: 24.05.2012 IT RM20120239
(43) Date of publication of application: 08.04.2015
(73) Proprietor: Glomeria Therapeutics S.r.l., 66100 Chieti (IT)
(72) Inventor: ARDUINI, Arduino, I-66100 Chieti (CH) (IT); ARRIZZA, Fabio, I-66100 Chieti (CH) (IT); CERASOLI, Paolo, I-66100 Chieti (CH) (IT)
(74) Representative: Leone, Mario
(86) International application number: PCT/IB2013/054051
(87) International publication number: WO 2013/175371

(56) References cited:
- EP-A1- 0 070 087
- WO-A1-2011/079226
- DE-A1-102005 020 764
- US-A- 3 944 261
- US-A1- 2006 260 699
- US-A1- 2009 143 723
- US-A1- 2010 030 194
- US-B1- 7 131 962

## Description

The present invention has as object a connection device, in particular for connecting one or more ends of respective ducts to that of another duct.

The invention also relates to the use of a catheter inside a living body, in particular a human body, in order to perform, through said catheter and through the ducts connected thereto, the introduction and removal of fluids into and from the living body.

A particular application example is constituted by the connection to a catheter inserted in a human body to perform the peritoneal dialysis treatment.

The known devices do not prevent with certainty the contact between the interchange fluids and the outer environment or they result to be difficult to be used by the user.

With reference to the peritoneal dialysis, in the first case a fluid contamination from outer, even pathogen agents may happen, which would involve a dangerous peritonitis, whereas in the second case, the user, often with limited cognitive and motor knowledge, it can make use mistakes and compromise the dialytic treatment itself. Furthermore, a complicated use may prevent from adopting such treatment on said subjects.

The document US 2006/260699 A describes a connection device for joining two ends of respective ducts to a third flexible duct. It comprises a casing receiving two nozzle-like terminal ends of a Y-shaped branch of the flexible duct and a connection member which must be engaged and disengaged on said casing, which has ending portions of the respective ducts shaped so as to implement the coupling and uncoupling of the nozzle-like terminal ends.

The technical problem underlying the present invention is to provide a connection device allowing to obviate the drawbacks mentioned with reference to the known art.

Such problem is solved by a connection device according to the enclosed claim 1.

The main advantage of the device according to the present invention environments with respect to the outer environment, by preventing possible contaminations from pathogen agents and by making the connection procedure easy to be performed.

Furthermore, with reference to the application in the peritoneal dialysis, the device according to the present invention reduces considerably the structural complexity of the disposable portion.

The present invention will be herein described according to a preferred embodiment example thereof, provided by way of example and not for limitative purposes with reference to the enclosed drawings wherein:

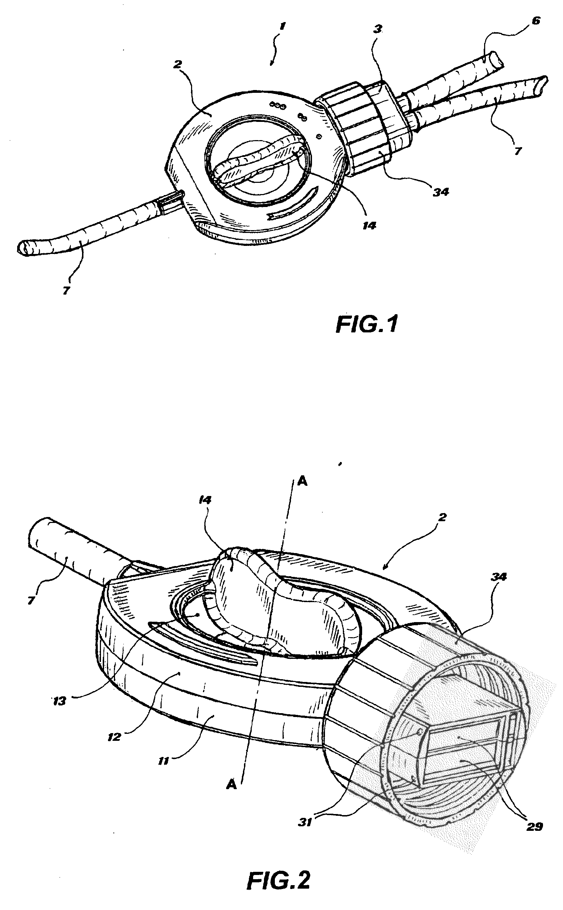

- figure 1 shows a perspective view of an embodiment example of a connection device according to the present invention;
- figure 2 shows a perspective view of a partial set of the connection device of figure 1;
- figure 3 shows a perspective view of a component of the connection device of figure 1;
- figure 4 shows an exploded perspective view of the connection device of figure 1;

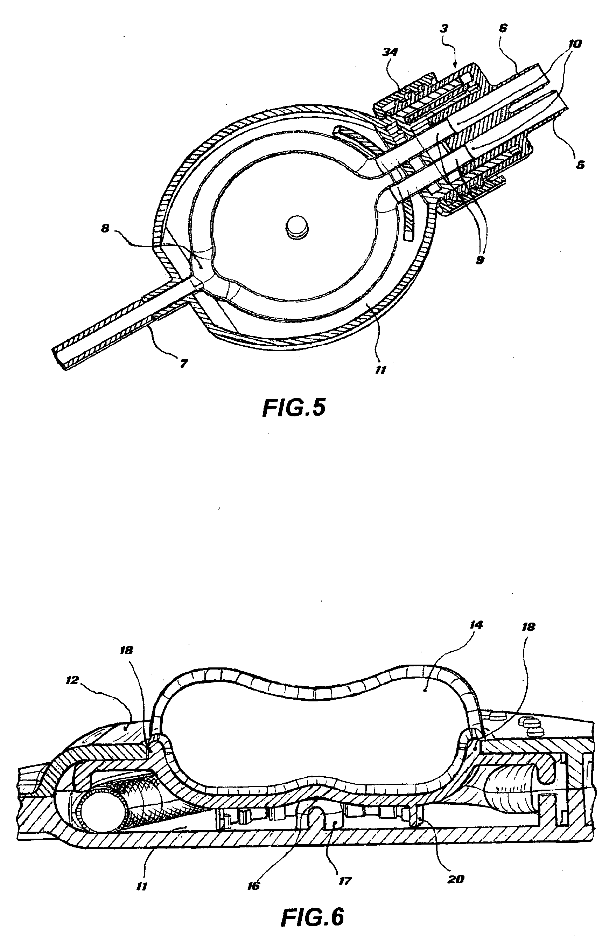

- figure 5 shows a perspective view in longitudinal section of the connection device of figure 1;
- figure 6 shows a perspective view in cross partial section of the connection device of figure 1;

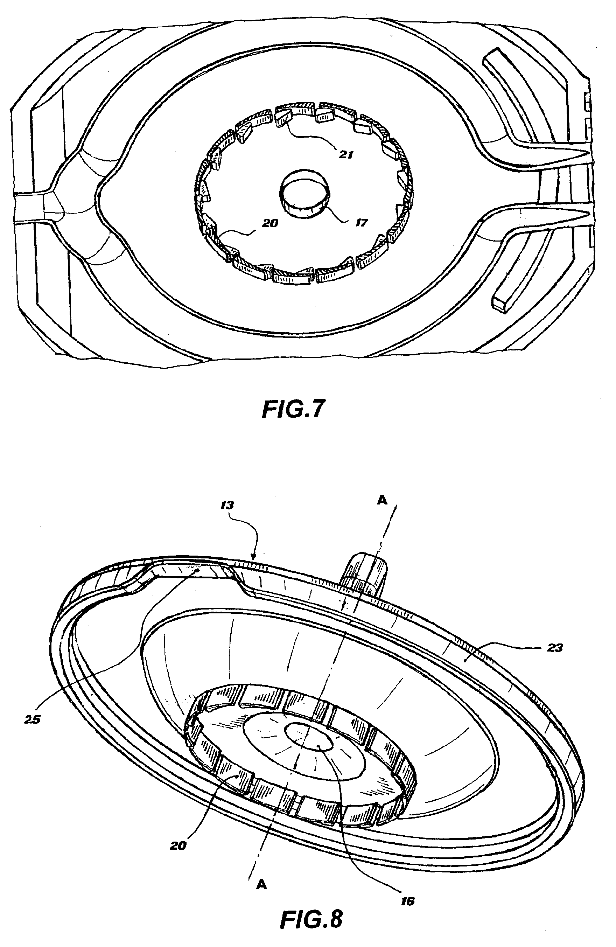

- figure 7 shows a perspective view of a detail in longitudinal partial section of the connection device of figure 1;
- figure 8 shows a perspective view of a component of the connection device of figure 1;

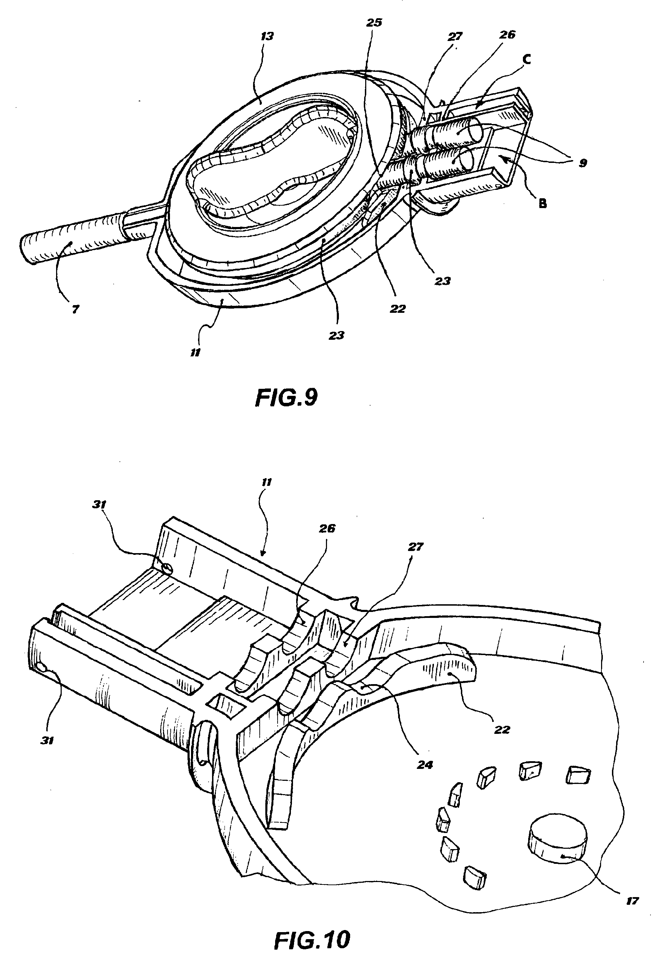

- figure 9 shows a perspective view of a partial set of the connection device of figure 1;
- figure 10 shows a perspective partial view of a component of the connection device of figure 1;

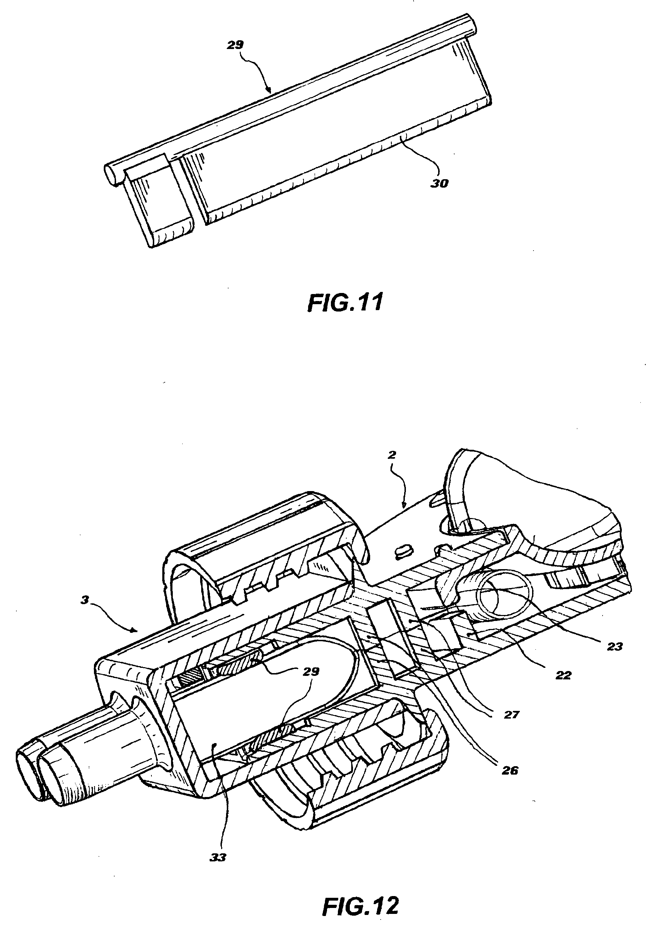

- figure 11 shows a perspective view of a component of the connection device of figure 1;
- figure 12 shows a perspective partial view in cross section of the connection device of figure 1;

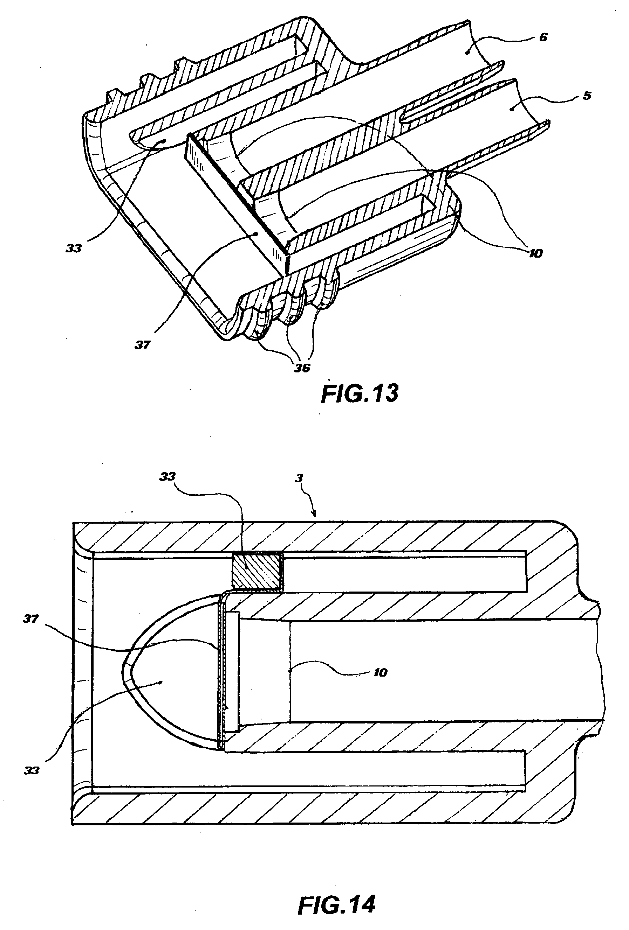

- figure 13 shows a perspective view in longitudinal section of a component of the connection device of figure 1;
- figure 14 shows a partial view in cross section of the device of a component of the connection device of figure 1;
- figure 15 shows a perspective partial view in cross section of the connection device of figure 1 in three subsequent operating positions; and
- figure 16 shows an additional partial perspective view in cross section of the connection device of figure 1 in three subsequent operating positions.

By referring to the figures, a connection device is designated as a whole with 1.

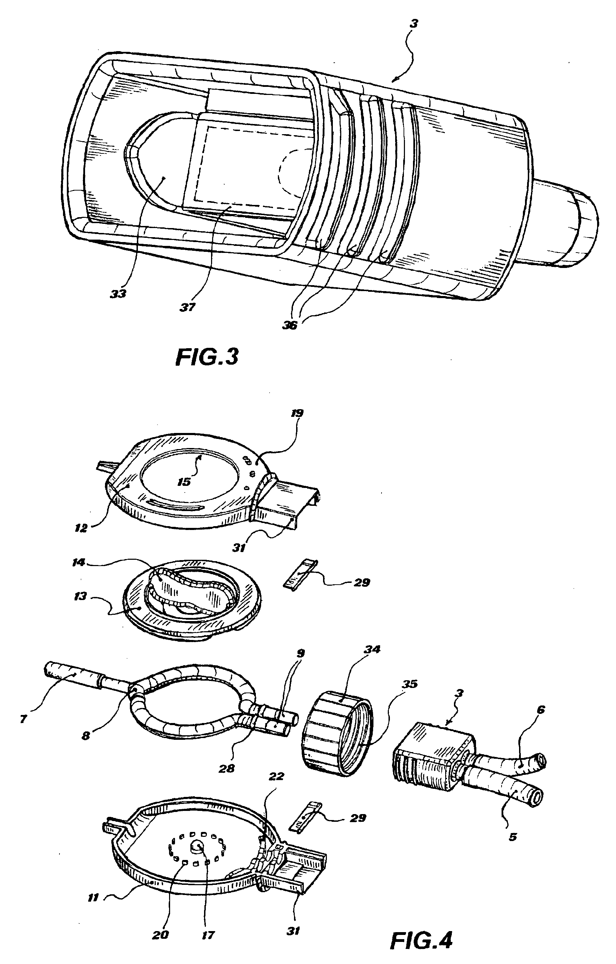

It is constituted by a closed container 2 approximately shaped like a round disk receiving a first flexible duct 7 and including a suitable y-shaped branch 8 thereof inside said container 2, which divides said duct into two duct sections arranged like a half-circle by following the shape of the round side walls of the container 2 (figure 4), each one having at its own distal end respective stiff terminal ends 9 forming an end nozzle.

On the opposite side of the inlet of the first duct 7 in the container 2, the device 1 has a removable connection member 3 enclosing the ending tubular portions 10 of two respective second and third duct 5 and 6.

Said ending tubular portions 10 are shaped like a truncated cone for the liquid-tight coupling with said nozzle-like terminal ends 9.

Said device 2 is constituted by two half-shells, respectively, a lower shell 11 and an upper shell 12, and by a rotor 13, constituting the plane upper wall of the upper half-shell 12, bearing a projecting knob 14 which can be actuated by the user and positioned inside a round opening 15 on the half-shell 12.

Said half-shells 11 e 12 are made integral therebetween during the assembly to form a closed casing 2.

Said rotor 13 is kept in seat by the contact between the lower portion thereof shaped like a spherical cap 16, with a pin 17 placed onto the lower half-shell 11, and the lower portion of the edge of said opening 15 with the upper portion of said rotor 13, as well as by the edge thereof 18, which allows only the rotation with respect to the axis A perpendicular to the container 2.

The actuation of the rotor 13 takes place, by the user of the device, by grasping and rotating said knob 14 with the fingers.

The rotor 13 can rotate in one single direction thanks to a mechanism which prevents the return thereof, implemented in the example shown herein by a first toothed profile 20 properly shaped and arranged on the inner face of the rotor 13 which is faced inwardly the container 2, with a plurality of small teeth apt to cooperate with a respective second toothed profile 21 formed on the bottom of the lower half-shell 11, so as to make the rotation of the rotor 13, with respect to the half-shells 11, 12 unidirectional and with a pre-fixed rotation angular pitch.

The rotation angular motion is further signalled to the user by a graduated scale 19 formed outside the upper half-shell 12, of the type with designation of phase I - II - III, preferably in relief for a possible tactile perception thereof.

The device 2 comprises a clogging system of the ducts 9 constituted by septa 22 and 23 aligned therebetween and placed respectively on the lower half-shell 11 and on the rotor 13.

The first one of said septa 22 projects vertically from the bottom of the lower half-shell 11 at said stiff terminal ends 9 and it is shaped to form two concave recesses 24 respectively apt to keep in seat the corresponding terminal end 9, whereas the second septum 23 is curved by projecting perpendicularly from the outer edge of the rotor towards the inside the container 2 and it is equipped with an indentation with a bevelled profile 25.

It results that, during the rotation of the rotor 13, implemented by the user through the knob 14, the nozzle-like terminal ends 9 are throttled by the second septum 23 except when the indentation 25 is at one of the respective terminal ends 9 in contrast with the first septum 22 which keeps them firm in their position. Therefore, the terminal ends 9 in this way result to be alternatively opened thanks to the elastic return of the flexible duct section thereof which brings back the walls thereof to a tubular shape.

The nozzle-like terminal ends 9 are further locked, axially and radially, inside the two half-shells 11 and 12 by positive connection between opposite profiles 26 and 27 shaped like a fork, symmetrically present on the half-shells 11 and 12 and respective radial thickenings 28 of said nozzle-like terminal ends 9.

The first flexible duct 7 and the related lines downwards the Y-shaped branch 8 are made integral to the lower half-shell 11 for example by means of gluing.

The distal end of the device 2 is divided into two separate areas: a first area B containing the nozzle-like terminal ends 9, and a second area C insulated from the first one, apt to receive inside thereof a projecting septum 33, shaped like a cusp, of the connection member 3.

Said terminal distal end of the device 2 is closed by two mobile walls 29 kept in position by means of a pin 30 and suitable holes 31 obtained on the half-shells 11 and 12.

The closing position of said walls 29 is guaranteed by two torsion springs anchored on the pin 30, not represented in the figures.

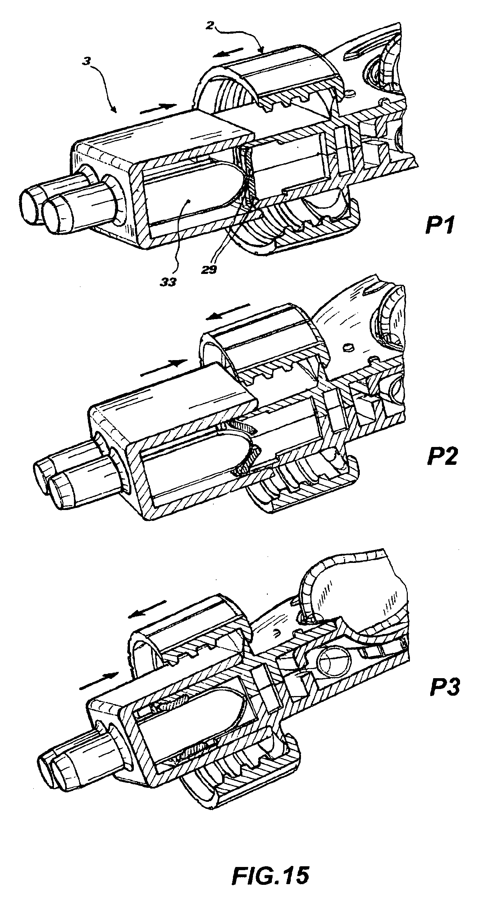

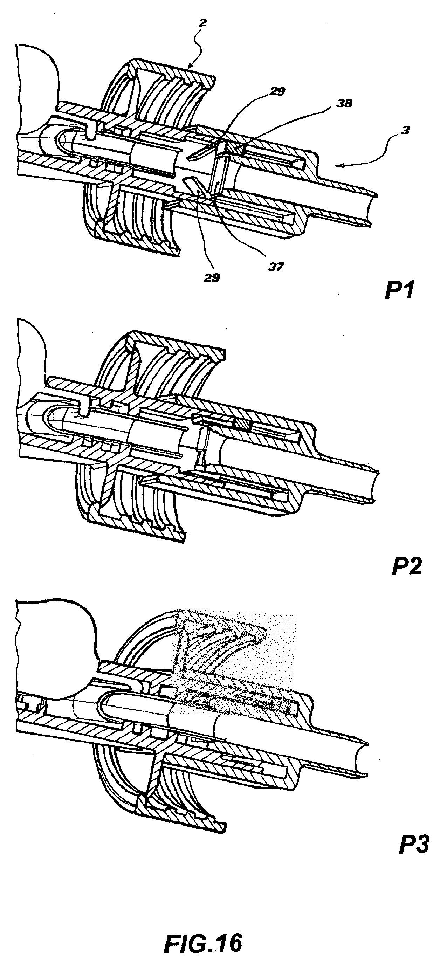

Said walls 29, by rotating towards the inside of the device 2 around the pin 30, open the distal end of the device 2 by making accessible from outside the two nozzle-like terminal ends 9. Such rotation takes place upon the connection between the closed container 2 and the removable connection member 3, by the effect of the contact between the cusp-like septum 33 and the mobile walls 29 according to the sequence P1, P2, P3 represented in figures 15 and 16.

The distal end of the device 2 is shaped to couple to the connection member 3 according to a guiding scheme with prismatic joint; such connection is made stable by a locking system which, in the present example, is constituted by a revolving ring nut 34 equipped with female screw 35 which intercepts the threads 36 obtained on the outer edge of the connection member 3.

The open section of the ending tubular portions 10 of the connection member 3 are protected respectively by a removable tear film 37, folded to form an edge ending with a thickening 38.

Upon inserting the connection member 3 in the closed container 2, such thickening 38 comes in contact with the edge of the upper half-shell 12 which drags it, thus causing the dragging and the consequent separation of said film 37 from the openings of the ending tubular portions 10, with the progressive opening, according to the sequence P1, P2, P3 of the respective second and third ducts 5, 6, which only subsequently connect to the nozzle-like terminal ends 9 of the first duct 7.

Therefore, the removable connection member 3 is of the non-reusable type and thus it can be classified as disposable.

With reference to the case of the peritoneal dialysis treatment, the device object of the present invention in the herein illustrated example is suitable to the use in the treatment called C.a.p.d. (Continuous Ambulatory Peritoneal Dialysis).

Such treatment takes place through inletting by gravity from an outer bag, by means of a suitable fixed catheter, a dialytic fluid into the peritoneal cavity. The dialytic fluid remains in the patient's abdomen for a few hours so as to allow the exchange of water and waste from blood to the dialytic liquid through the peritoneal membrane. At the end of the treatment the fluid is discharged into a suitable empty bag.

In particular in the present example, the first duct 7 is connected to the patient's peritoneum and the second and third ducts 5 and 6 are respectively connected to the bag full of peritoneal solution, said loading bag, and to the empty bag discharging the dialytic liquid. Such treatment is repeated several times a day.

In the not-use intervals, between one treatment and the other one, the device 2 is in the initial configuration signalled with I on the graduated scale existing on the upper half-shell 11.

The ducts downwards of the Y-shaped branch 8 of the first duct 7 ending in the the nozzle-like terminal ends 9 are both closed by a clogging system inside the closed container 2; the walls 29 protect the nozzle-like terminal ends 9 of the first duct 7.

The insertion of the removable connection member 3 in the distal end of the closed container 2 brings in contact, inside the area C, the profile 33 with the mobile walls 29 and it controls the opening thereof inside the area B insulated with respect to the outer environment.

Subsequently, the thickening 38 of the removable tear film 37 is in contact by interference with the edge of the upper half-shell 12 and it is dragged thereby, by ungluing the film 37 until uncovering ending tubular portions 10 of two ducts 5, 6.

In the following phase, by continuing the insertion of the removable member 3 with the ending portion of the closed container 2, the fluid-tight connection between the nozzle-like terminal ends 9 of the first duct 7 and the ending tubular portions 10 of the second and third duct 5, 6 takes place, by making possible the exchange of the dialytic fluid between the bags and the peritoneum.

The connection between the closed container 2 and the removable connection member 3 is performed by the user by rotating the ring nut 34, the female screw thereof is placed in engagement with the threads 36 outside the removable connection member 3.

The user rotates the knob 14 as far as the angular position designated with II with reference to the graduated scale 19, only the first one of the nozzle-like terminal ends 9 connected to the loading bag opens; the user breaks a suitable (not represented) fracture cone placed on the second duct 5 by allowing the dialytic solution flow towards the peritoneum.

In the subsequent phase, the user rotates again the knob as far as the position III with reference to the graduated scale 19. In this configuration, only the second one of the terminal ends 9 results to be opened to allow the dialytic solution flow from the peritoneum to the discharging bag through the third duct 6, possibly equipped too with specific fracture cone.

At the end of the dialysis treatment, an additional rotation of the rotor 13 through the knob 14 as far as the position signalled with I with reference to the graduated scale 19 and the subsequent disconnection of the removable connection member 3 from the closed container 2 restores the initial configuration condition.

Thereafter the so-described cycle can be repeated by using a new removable connection device 3.

A connection device as described in the present invention can be implemented by a person skilled in the art in a configuration suitable to the peritoneal dialysis treatment called A.p.d. (Automatized Peritoneal Dialysis) wherein the inletting and the removal of the dialytic fluid are performed by a suitable Cycler machine.

To the above described connection devices a person skilled in the art, in order to satisfy additional and contingent needs, can introduce several additional modifications and variants, all however within the protective scope of the present invention, as defined by the enclosed claims.

## Claims

1. A connection device (1) to connect two ends of respective ducts (5, 6) to another flexible duct (7) comprising:
• a closed container (2) containing two nozzle-like terminal ends (9) of a Y-shaped branch (8) of a flexible duct (7);
• a removable connection member (3) apt to be engaged/disengaged on said closed container (2), containing the ending portions of the respective ducts (5, 6) shaped to couple/uncouple with said nozzle-like terminal ends (9), a distal end of the container (2) and the connecting member (3) being shaped to couple according to a guiding scheme with prismatic joint,
**characterised in that** it further comprises:
• a closure with mobile walls (29) on said closed container (2) actuated in opening and closing by the contact of a projecting septum (33) of said connection member (3) upon the connection/disconnection between said closed container (2) and said connection member (3); and
• a closure with removable tear film (37) arranged at the ending portions (10) of said ducts (5, 6) to close the respective opened sections inside said connection member (3), apt to be removed from the interference between the edge of said closed container (2) and a thickened edge thereof (38) upon the connection between said closed container (2) and said connection member (3),
the connection of said closed container (2) and said connection member (3) determining the engagement and the disengagement of said nozzle-like terminal ends (9) with said ending portions of respective ducts (5, 6).

2. The connection device (1) according to claim 1, wherein said closed container (2) comprises two respectively lower (11) and upper (12) half-shells one thereof is equipped with a slot (15) wherein a rotor (13) is inserted outwardly shaped to form a knob (14).

3. The connection device according to claim 1, wherein said flexible duct (7) and the related ducts downwards said Y-shaped branch (8) are integral with said lower half-shell (11).

4. The connection device according to claims 1 and 2, wherein said half-shells (11, 12) include shaped profiles (26, 27) to lock axially and radially said nozzle-like terminal ends (9) of said flexible duct (7).

5. The connection device according to claims 1 and 2, wherein said half-shells are shaped in the distal end connection of the container (2) to form two separated areas (B, C) one containing said nozzle-like terminal ends (9) of said flexible duct (7), the other one suitable to receive a shaped septum (33) of said removable connection member (3).

6. The connection device according to claims 1 and 2, wherein said closed container (2) comprises mobile walls (29) hinged in rotation respectively through a pin (30) inserted in suitable holes on said half-shells (11, 12) and respectively equipped with a return spring member in the closing position.

7. The connection device according to claim 6, wherein said shaped septum (33), during the connection/disconnection, acts as actuation by contact for opening and closing said mobile walls (29).

8. The connection device according to claim 1, wherein said removable connection member (3) includes the ending portions (10) of said ducts (5, 6) shaped in suitable way for the liquid-tight coupling with said nozzle-like terminal ends (9).

9. The connection device according to the previous claims, wherein the ending portions of said closed container (2) and said removable connection member (3) are shaped to couple according to the guiding scheme with prismatic joint in a way suitable to perform in sequence:
• the connection thereof to form inside thereof a closed volume insulated from the outer environment;
• the opening of said mobile walls (29); and
• the removal of said removable tear film (37),
• the connection of said nozzle-like terminal ends (9) of said flexible duct (7) with said ending portions (10) of two ducts (5, 6).

10. The connection device according to claim 1, wherein a fastening system makes integral said closed container (2) and said removable connection member (3) upon the connection thereof.

11. The connection device (1) according to claims 1 and 2, wherein said closed container (13) comprises a seat obtained on said lower and upper half-shells (11, 12) constraining said rotor (13) to the rotation only, which can be carried out by the user by means of said knob (14).

12. The device according to claim 11, wherein said closed container (2) comprises a system which makes the rotation of said rotor (13) unidirectional with fixed pitch.

13. The device according to claim 12, wherein the position of said rotor (13) equipped with said knob (14) is designated by means of a graduated scale (19) placed outside the closed container (2).

14. The device according to claims 1 and 2, wherein said closed container (2) comprises an alternative clogging system of the ducts downwards of said Y-shaped branch (8) of said flexible duct (7) constituted by septa (22, 23) respectively aligned therebetween on said lower half-shell (11), shaped to keep in seat said ducts, and on said rotor (13) shaped so as to throttle or not the ducts downwards said Y-shaped branch (8) of said flexible duct (7) depending upon the related angular position between said rotor (13) and said lower shell (11).

15. Disposable kit for treating the peritoneal dialysis, which comprises a connection device (1) according to any one of the previous claims, a bag containing a suitable dialysis solution, an empty bag and the related connection ducts for the dialysis of C.a.p.d. type.

16. Disposable kit for treating the peritoneal dialysis, which comprises a connection device (1) according to any one of the claims 1 to 15, and the related connection ducts or pumping devices for the dialysis of A.p.d. type.

## Patentansprüche

1. Eine Verbindungsvorrichtung (1) zur Verbindung von zwei Enden jeweiliger Kanäle (5, 6) an eine andere flexible Leitung (7), umfassend:
• einen geschlossenen Behälter (2) mit zwei düsenartigen Anschlußenden (9) einer Y-förmigen Verzweigung (8) einer flexiblen Rohrleitung (7);
• ein abnehmbares Verbindungselement (3) das geeignet ist, um an dem geschlossenen Behälter (2) in Eingriff gebracht zu werden / davon gelöst zu werden, umfassend die Endbereiche der jeweiligen Kanäle (5, 6), die geformt sind, um mit den düsenartigen Anschlussenden (9) zu koppeln / zu entkoppeln, wobei ein distales Ende des Behälters (2) und das Verbindungselement (3) geformt sind, um gemäß einem Führungsschema mit prismatischer Verbindung zu koppeln;
**dadurch gekennzeichnet, dass** die Verbindungsvorrichtung weiterhin umfasst:
• einen Verschluss mit beweglichen Wänden (29) an dem geschlossenen Behälter (2) die zum Öffnen und Verschließen durch den Kontakt einer vorstehenden Wand (33) des Verbindungselementes (3) bei der Verbindung / Trennung zwischen dem geschlossenen Behälter (2) und dem Verbindungselement (3) betätigt werden; und
• einen Verschluss mit entfernbarem Reißfilm (37), der an den Endbereichen (10) der Kanäle (5, 6) zum Verschließen der jeweiligen geöffneten Abschnitte innerhalb des Verbindungselements (3) angeordnet ist und ausgelegt ist um von dem Eingriff zwischen der Kante des geschlossenen Behälters (2) und einer verdickten Kante davon (38) bei Verbindung zwischen dem geschlossenen Behälter (2) und dem Verbindungselement (3) entfernt zu werden,
wobei die Verbindung des geschlossenen Behälters (2) und des Verbindungselementes (3) den Eingriff und das Lösen der düsenartigen Anschlußenden (9) mit den Endabschnitten der jeweiligen Kanäle (5, 6) bestimmt.

2. Verbindungsvorrichtung (1) nach Anspruch 1, wobei der geschlossene Behälter (2) zwei jeweils untere (11) und obere (12) Halbschalen umfasst, wobei eine davon mit einem Schlitz (15) ausgestattet ist, in den ein Rotor (13) eingebracht ist, der so nach außen geformt ist um einen Knopf (14) zu bilden.

3. Verbindungsvorrichtung nach Anspruch 1, wobei die flexible Leitung (7) und die zugehörigen Kanäle abwärts der Y-förmigen Verzweigung (8) integral mit der unteren Halbschale (11) sind.

4. Verbindungsvorrichtung nach den Ansprüchen 1 und 2, wobei die Halbschalen (11, 12) geformte Profile (26, 27) zum axialen und radialen Arretieren der düsenartigen Anschlußenden (9) der flexiblen Leitung (7) umfassen.

5. Verbindungsvorrichtung nach den Ansprüchen 1 und 2, wobei die Halbschalen in der distalen Ende-Verbindung des Behälters (2) geformt sind, um zwei getrennte Bereiche (B, C) zu bilden, wobei einer die düsenartigen Anschlußenden (9) der flexiblen Leitung (7) enthält und der andere geeignet ist, um eine geformte Wand (33) des abnehmbaren Verbindungselementes (3) aufzunehmen.

6. Verbindungsvorrichtung nach den Ansprüchen 1 und 2, wobei der geschlossene Behälter (2) bewegliche Wände (29) aufweist, die drehbar gelagert sind jeweils durch einen Stift (30), der in geeignete Löcher an den Halbschalen (11, 12) eingebracht ist, und jeweils ausgestattet mit einem Rückfederelement in die Schließstellung.

7. Verbindungsvorrichtung nach Anspruch 6, wobei die geformte Wand (33) während der Verbindung / Trennung als Betätigung durch Kontakt zum Öffnen und Schließen der beweglichen Wände (29) wirkt.

8. Verbindungsvorrichtung nach Anspruch 1, wobei das abnehmbare Verbindungselement (3) die Endbereiche (10) der Kanäle (5, 6) aufweist, die in geeigneter Weise für die flüssigkeitsdichte Kupplung mit den düsenartigen Anschlussenden (9) geformt sind.

9. Verbindungsvorrichtung nach den vorhergehenden Ansprüchen, wobei die Endbereiche des geschlossenen Behälters (2) und des abnehmbaren Verbindungselementes (3) geformt sind, um entsprechend dem Führungsschema mit prismatischer Verbindung in einer geeigneten Weise zu koppeln, um in Abfolge auszuführen :
• die Verbindung davon, um im Inneren davon ein geschlossenes Volumen zu bilden, das von der äußeren Umgebung isoliert ist;
• die Öffnung der beweglichen Wände (29); und
• das Entfernen des entfernbaren Reißfilms (37),
• die Verbindung der düsenartigen Anschlußenden (9) der flexiblen Leitung (7) mit den Endbereichen (10) von zwei Leitungen (5, 6).

10. Verbindungsvorrichtung nach Anspruch 1, wobei ein Befestigungssystem den geschlossenen Behälter (2) und das entfernbare Verbindungselement (3) bei deren Verbindung integral macht.

11. Verbindungsvorrichtung (1) nach den Ansprüchen 1 und 2, wobei der geschlossene Behälter (13) einen Sitz umfasst, der an den unteren und oberen Halbschalen (11, 12) erhalten wird und den Rotor (13) nur auf die Drehung beschränkt, die durch den Benutzer mittels des Knopfes (14) durchgeführt werden kann.

12. Vorrichtung nach Anspruch 11, wobei der geschlossene Behälter (2) ein System umfasst, das die Drehung des Rotors (13) unidirektional mit festen Stufen macht.

13. Vorrichtung nach Anspruch 12, wobei die Position des Rotors (13), der mit dem Knopf (14) ausgestattet ist, mittels einer graduierten Skala (19) bezeichnet ist, die außerhalb des geschlossenen Behälters (2) angeordnet ist.

14. Vorrichtung nach den Ansprüchen 1 und 2, wobei der geschlossene Behälter (2) ein alternatives Blockiersystem der Kanäle abwärts der Y-förmigen Verzweigung (8) der flexiblen Leitung (7) umfasst, das durch Wände(22, 23) gebildet ist, die jeweils dazwischen auf der unteren Halbschale (11) ausgerichtet sind und geformt sind, um die Kanäle im Sitz zu halten, und auf dem Rotor (13) so geformt sind, um die Kanäle abwärts der Y-förmigen Verzweigung (8) der flexiblen Leitung (7) zu drosseln oder nicht, in Abhängigkeit von der zugehörigen Winkelposition zwischen dem Rotor (13) und der unteren Schale (11).

15. Einweg-Kit für die Behandlung der Peritonealdialyse, die umfasst eine Verbindungsvorrichtung (1) nach einem der vorhergehenden Ansprüche, einen Beutel enthaltend eine geeigneten Dialyselösung, einen leeren Beutel und die zugehörigen Verbindungskanäle für die Dialyse des C.a.p.d. Typs.

16. Einweg-Kit für die Behandlung der Peritonealdialyse, die eine Verbindungsvorrichtung (1) nach einem der Ansprüche 1 bis 15 und die zugehörigen Verbindungskanäle oder Pumpvorrichtungen für die Dialyse des A.p.d. Typs umfasst.

## Revendications

1. Dispositif de raccordement (1) pour raccorder deux extrémités de conduites (5, 6) respectives à une autre conduite souple (7), comportant :
• un conteneur fermé (2) contenant deux extrémités terminales analogues à des buses (9) d'un embranchement en forme de Y (8) d'une conduite souple (7),
• un élément de raccordement amovible (3) apte à être engagé sur ledit conteneur fermé (2) et désengagé de celui-ci, contenant les parties d'extrémité des conduites (5, 6) respectives mises en forme pour un couplage avec lesdites extrémités terminales analogues à des buses (9) ou une séparation de celles-ci, une extrémité distale du conteneur (2) et de l'élément de raccordement (3) étant mise en forme pour un couplage suivant un schéma de guidage avec une articulation prismatique,
**caractérisé en ce qu'**il comporte en outre :
• une fermeture pourvue de parois mobiles (29) sur ledit conteneur fermé (2), actionnée en ouverture et en fermeture par le contact d'un septum saillant (33) dudit élément de raccordement (3) lors du raccordement entre ledit conteneur fermé (2) et ledit élément de raccordement (3) et de la séparation de ceux-ci, et
• une fermeture pourvue d'un film déchirable amovible (37) disposé sur les parties d'extrémité (10) desdites conduites (5, 6) pour fermer les sections ouvertes respectives à l'intérieur dudit élément de raccordement (3), apte à être retiré de l'interférence entre le bord dudit conteneur fermé (2) et un bord épaissi de celui-ci (38) lors du raccordement entre ledit conteneur fermé (2) et ledit élément de raccordement (3),
le raccordement dudit conteneur fermé (2) et dudit élément de raccordement (3) déterminant l'engagement et le désengagement desdites extrémités terminales analogues à des buses (9) avec lesdites parties d'extrémité des conduites (5, 6) respectives.

2. Dispositif de raccordement (1) selon la revendication 1, dans lequel ledit conteneur fermé (2) comporte deux demies coques inférieure (11) et supérieure (12) respectivement, l'une de celles-ci est équipée d'une fente (15) dans laquelle un rotor (13) est inséré vers l'extérieur mis en forme de manière à former un bouton (14).

3. Dispositif de raccordement selon la revendication 1, dans lequel ladite conduite souple (7) et les conduites associées vers le bas dudit embranchement en forme de Y (8) sont solidaires de ladite demie coque inférieure (11).

4. Dispositif de raccordement selon les revendications 1 et 2, dans lequel lesdites demies coques (11, 12) incluent des profils conformés (26, 27) pour bloquer axialement et radialement lesdites extrémités terminales analogues à des buses (9) de ladite conduite souple (7).

5. Dispositif de raccordement selon les revendications 1 et 2, dans lequel lesdites demies coques sont mises en forme dans le raccordement d'extrémité distale du conteneur (2) de manière à former deux zones séparées (B, C), l'une contenant lesdites extrémités terminales analogues à des buses (9) de ladite conduite souple (7), l'autre étant adaptée pour recevoir un septum conformé (33) dudit élément de raccordement amovible (3).

6. Dispositif de raccordement selon les revendications 1 et 2, dans lequel ledit conteneur fermé (2) comporte des parois mobiles (29) articulées en rotation respectivement par un ergot (30) inséré dans des trous adaptés sur lesdites demies coques (11, 12) et respectivement équipées d'un élément à ressort de rappel dans la position de fermeture.

7. Dispositif de raccordement selon la revendication 6, dans lequel ledit septum conformé (33), pendant le raccordement/la séparation, agit par actionnement par contact pour ouvrir et fermer lesdites parois mobiles (29).

8. Dispositif de raccordement selon la revendication 1, dans lequel ledit élément de raccordement amovible (3) inclut les parties d'extrémité (10) desdites conduites (5, 6) mises en forme de manière adaptée pour un couplage étanche aux liquides avec lesdites extrémités terminales analogues à des buses (9).

9. Dispositif de raccordement selon les revendications précédentes, dans lequel les parties d'extrémité dudit conteneur fermé (2) et dudit élément de raccordement amovible (3) sont conformées pour un couplage suivant le schéma de guidage avec une articulation prismatique d'une manière adaptée pour réaliser dans l'ordre :
• le raccordement de celles-ci pour former à l'interne de celles-ci un volume fermé isolé de l'environnement extérieur,
• l'ouverture desdites parois mobiles (29), et
• le retrait dudit film déchirable amovible (37),
• le raccordement desdites extrémités terminales analogues à des buses (9) de ladite conduite souple (7) avec lesdites parties d'extrémité (10) des deux conduites (5, 6).

10. Dispositif de raccordement selon la revendication 1, dans lequel un système de fixation rend solidaires ledit conteneur fermé (2) et ledit élément de raccordement amovible (3) lors du raccordement de ceux-ci.

11. Dispositif de raccordement (1) selon les revendications 1 et 2, dans lequel ledit conteneur fermé (2) comporte un appui obtenu sur lesdites demies coques inférieure et supérieure (11, 12) contraignant ledit rotor (13) à la rotation uniquement, qui peut être exécutée par l'utilisateur au moyen dudit bouton (14).

12. Dispositif selon la revendication 11, dans lequel ledit conteneur fermé (2) comporte un système qui rend la rotation dudit rotor (13) unidirectionnelle avec un pas fixe.

13. Dispositif selon la revendication 12, dans lequel la position dudit rotor (13) équipé dudit bouton (14) est désignée au moyen d'une échelle graduée (19) placée à l'extérieur du conteneur fermé (2).

14. Dispositif selon les revendications 1 et 2, dans lequel ledit conteneur fermé (2) comporte un système de colmatage alternatif des conduites vers le bas dudit embranchement en forme de Y (8) de ladite conduite souple (7), constitué par des septums (22, 23) respectivement alignés entre celles-ci sur ladite demie coque inférieure (11), mis en forme de manière à maintenir en appui lesdites conduites, et sur ledit rotor (13) mis en forme de manière à étrangler ou non les conduites vers le bas dudit embranchement en forme de Y (8) de ladite conduite souple (7) en fonction de la position angulaire associée entre ledit rotor (13) et ladite demie coque inférieure (11).

15. Kit jetable pour traiter la dialyse péritonéale, lequel comporte un dispositif de raccordement (1) selon l'une quelconque des revendications précédentes, une poche contenant une solution de dialyse adaptée, une poche vide et les conduites de raccordement associées pour la dialyse de type dialyse péritonéale ambulatoire continue (C.a.p.d).

16. Kit jetable pour traiter la dialyse péritonéale, lequel comporte un dispositif de raccordement (1) selon l'une quelconque des revendications 1 à 15, et les conduites de raccordement associées ou des dispositifs de pompage pour la dialyse de type dialyse péritonéale ambulatoire (A.p.d.).
